(19) Europäisches Patentamt
European Patent Office
Office européen des brevets

(11) **EP 4 180 462 A1**

(12) **EUROPEAN PATENT APPLICATION**
published in accordance with Art. 153(4) EPC

(43) Date of publication:
**17.05.2023 Bulletin 2023/20**

(21) Application number: **21838876.7**

(22) Date of filing: **09.07.2021**

(51) International Patent Classification (IPC):
*C08B 15/04* (2006.01)    *A61Q 1/00* (2006.01)
*A61Q 19/00* (2006.01)    *A61K 9/10* (2006.01)
*A61K 8/73* (2006.01)    *A61K 47/38* (2006.01)

(52) Cooperative Patent Classification (CPC):
**A61K 8/73; A61K 9/10; A61K 47/38; A61Q 1/00; A61Q 19/00; C08B 15/04**

(86) International application number:
**PCT/JP2021/025936**

(87) International publication number:
**WO 2022/009979 (13.01.2022 Gazette 2022/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.07.2020 JP 2020118641**

(71) Applicant: **Toagosei Co., Ltd.**
**Tokyo, 105-0003 (JP)**

(72) Inventors:
• **MATSUKI, Shiroshi**
**Nagoya-shi, Aichi 455-0026 (JP)**

• **TSUIKI, Toshihiko**
**Nagoya-shi, Aichi 455-0026 (JP)**
• **TAKADA, Jun**
**Nagoya-shi, Aichi 455-0026 (JP)**
• **KAYANO, Hidenari**
**Nagoya-shi, Aichi 455-0026 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **OXIDIZED CELLULOSE, NANOCELLULOSE, AND DISPERSIONS THEREOF**

(57)    The present invention provides an oxidized cellulose that exhibits an excellent fibrillatability and is an oxide of a cellulose raw material by a hypochlorous acid or a salt thereof. The oxidized cellulose substantially does not contain a N-oxyl compound and has a degree of polymerization of not more than 600.

EP 4 180 462 A1

## Description

## Technical Field

The present invention relates to oxidized cellulose, nanocellulose, and their dispersions. More particularly, the present invention relates to an oxidized cellulose provided by the oxidation of a cellulose raw material with an oxidant, to an oxidized cellulose dispersion comprising this oxidized cellulose, to nanocellulose provided by the fibrillation of this oxidized cellulose, and to a nanocellulose dispersion comprising this nanocellulose.

## Background Art

Various art has been proposed with regard to producing nanocellulose materials, e.g., cellulose nanofiber (also referred to as "CNF" in the following), by the oxidation of various types of cellulose raw materials with an oxidant and the microfine-sizing of the resulting oxidized cellulose (refer, for example, to Patent Document 1 and Patent Document 2).

Patent Document 1 discloses the production of oxidized cellulose by the oxidation of a cellulose raw material using a hypochlorous acid or a salt thereof as the oxidant at high concentration conditions of a 14 to 43 mass% available chlorine concentration in the reaction system. Patent Document 2 discloses the production of oxidized cellulose by the oxidation of a cellulose raw material while adjusting the pH to 5.0 to 14.0 and using a hypochlorous acid or a salt thereof as the oxidant and using an available chlorine concentration in the reaction system of 6 to 14 mass%. In this art, the oxidation process is carried out without using an N-oxyl compound, e.g., 2,2,6,6-tetramethyl-1-piperidine-N-oxy radical (TEMPO), as the catalyst, and as a consequence an N-oxyl compound does not present in the cellulose fiber and it thus becomes possible to produce a nanocellulose material while devising a reduction in the effects on, e.g., the environment.

Citation List

Patent Documents

Patent Document 1: WO 2018/230354

Patent Document 2: WO 2020/027307

## Summary

Technical Problem

Patent Document 1 and Patent Document 2 disclose, as specific examples of the production of nanocellulose materials by a microfine-sizing of oxidized cellulose, examples in which the nanocellulose material is obtained via a fibrillation step using a mechanical treatment using an ultrasound homogenizer. However, there is additional room for improvement in this treatment with regard to the energy required for fibrillation. Viewed from the perspective of production costs, the production of nanocellulose materials requires an easily fibrillatable oxidized cellulose that enables fibrillation to be carried out even under mild treatment conditions. In addition, the oxidized cellulose residing in the state prior to fibrillation into a nanocellulose material must exhibit a good fibrillatability in order to carry out a stable production of microfine-sized cellulose fiber, or in order to obtain a highly transparent nanocellulose material that exhibits little light scattering in dispersion media.

The present invention was pursued in view of these circumstances, and the main object of the present invention is to provide an oxidized cellulose that exhibits an excellent fibrillatability.

Solution to Problem

The present invention provides the following means in order to solve the aforementioned problem.

[1] An oxidized cellulose that is an oxide of a cellulose raw material by a hypochlorous acid or a salt thereof, wherein the oxidized cellulose substantially does not comprise a N-oxyl compound and has a degree of polymerization of not more than 600.

[2] The oxidized cellulose according to [1], wherein the oxidized cellulose has a carboxy group content of 0.30 to 2.0 mmol/g.

[3] The oxidized cellulose according to [1] or [2], wherein an aqueous nanocellulose dispersion obtained by carrying out a fibrillation treatment on an aqueous dispersion of said oxidized cellulose present at a concentration of 0.1 mass% using a planetary centrifugal mixer and conditions of 10 minutes, a revolution rate of 2,000 rpm, and a rotation rate of 800 rpm, has a light transmittance of at least 60%.

[4] An oxidized cellulose that is an oxide of a cellulose raw material, wherein an aqueous nanocellulose dispersion obtained by carrying out a fibrillation treatment on an aqueous dispersion of said oxidized cellulose present at a concentration of 0.1 mass% using a planetary centrifugal mixer and conditions of 10 minutes, a revolution rate of 2,000 rpm, and a rotation rate of 800 rpm, has a light transmittance of at least 60%.

[5] The oxidized cellulose according to [4], wherein the oxidized cellulose is an oxide of a cellulose raw material by a hypochlorous acid or a salt thereof.

[6] An oxidized cellulose dispersion in which the oxidized cellulose according to any of [1] to [5] is dispersed in a dispersion medium.

[7] A nanocellulose having an average fiber width of 1 to 200 nm and provided by the fibrillation of the oxidized cellulose according to any of [1] to [6].

[8] A nanocellulose obtained by subjecting the oxidized cellulose according to any of [1] to [6] to a fibrillation treatment for 3 to 15 minutes using a planetary centrifugal mixer at a revolution rate of 1,200 to 2,500 rpm and a rotation rate of 600 to 1,000 rpm.

[9] A nanocellulose dispersion in which the nanocellulose according to [7] or [8] is dispersed in a dispersion medium.

Advantageous Effects of Invention

An oxidized cellulose that exhibits an excellent fibrillatability can be obtained in accordance with the present invention. In particular, even when the fibrillation treatment is carried out under mild conditions, the oxidized cellulose according to the present invention can be uniformly microfine-sized and exhibits an excellent ease-of-fibrillatability.

**Description of Embodiments**

<<The oxidized cellulose>>

The oxidized cellulose according to the present disclosure (also referred to hereafter as the "present oxidized cellulose") is a fibrous cellulose provided by the oxidation of a cellulose raw material with a hypochlorous acid or a salt thereof, and is an oxidized cellulose prior to the execution thereon of a fibrillation treatment. The present oxidized cellulose can also be referred to as the oxide of a cellulose raw material by a hypochlorous acid or a salt thereof. Cellulose is the main component of plants, and bundles of cellulose molecules are referred to as cellulose microfibrils. The cellulose in the cellulose raw material is also included in the state of this cellulose microfibrillar.

In addition, the "oxidized cellulose", because it is a fibrous cellulose provided by oxidation as indicated above, is also referred to as "oxidized cellulose fiber".

The oxidized cellulose substantially does not comprise a N-oxyl compound because the cellulose raw material is oxidized with a hypochlorous acid or a salt thereof. In this Description, "substantially does not comprise a N-oxyl compound" means that either a N-oxyl compound is entirely absent from the oxidized cellulose or the content of a N-oxyl compound, with reference to the total amount of the oxidized cellulose, is not more than 2.0 mass-ppm, with not more than 1.0 mass-ppm being preferred. In addition, "substantially does not comprise a N-oxyl compound" also refers to the case in which the N-oxyl compound content, as an increment from the cellulose raw material, is preferably not more than 2.0 mass-ppm and more preferably not more than 1.0 mass-ppm.

By having the oxidized cellulose be substantially free of N-oxyl compounds, the residual presence of N-oxyl compounds, which are concerning with regard to their impact on the environment and the human body, in the oxidized cellulose can be inhibited. The N-oxyl compound content can be measured by a known means. Methods that use a total trace nitrogen analyzer are an example of a known means. Specifically, the N-oxyl compound-derived nitrogen component in the oxidized cellulose can be measured as the amount of nitrogen using a total trace nitrogen analyzer (for example, instrument name: TN-21 00H, from Mitsubishi Chemical Analytech Co., Ltd.).

The present oxidized cellulose is particularly described in the following.

[The degree of polymerization]

In a favorable embodiment of the present disclosure, the degree of polymerization of the present oxidized cellulose is not more than 600. When the degree of polymerization of the present oxidized cellulose exceeds 600, a trend appears of requiring large amounts of energy for fibrillation and a trend appears of being unable to develop a satisfactory ease-of-fibrillatability. Moreover, when the degree of polymerization of the present oxidized cellulose exceeds 600, unsatisfactorily fibrillated oxidized cellulose then becomes prominent, and as a result, when nanocellulose provided by microfine-sizing this is dispersed in a dispersion medium, inter alia, light scattering becomes prominent and the transparency is reduced. Variability is also produced in the size of the obtained nanocellulose and a tendency for the quality to be nonuniform occurs. As a consequence, the viscosity of a slurry containing the nanocellulose along with solid particles (also referred to hereafter as a "nanocellulose-containing slurry") will be unstable, and the handling characteristics and coating characteristics of the slurry may also be impaired. From the perspective of ease-of-fibrillatability, there are no specific limitations on the lower limit of the degree of polymerization of the present oxidized cellulose. However, when the degree of polymerization of the present oxidized cellulose is less than 50, the proportion of particulate cellulose becomes larger than that of fibrous cellulose and the quality of the slurry becomes nonuniform and the viscosity becomes unstable; in addition, the ability to obtain a thixotropic behavior, which is a characteristic feature of nanocelluloses, is impaired. Viewed from these perspectives, the degree of polymerization of the present oxidized cellulose is preferably 50 to 600.

The degree of polymerization of the present oxidized cellulose is more preferably not more than 580, still more preferably not more than 560, even more preferably not more than 550, still more preferably not more than 500, still more preferably not more than 450, and still more preferably not more than 400. From the perspective of providing a good viscosity stability and coatability for the slurry, the lower limit for the degree of polymerization is more preferably at least 60, still more preferably at least 70, even more preferably at least 80, still more preferably at least 90, still more preferably at least 100, still more preferably at least 110, and still more preferably at least 120. Preferred ranges for the degree of polymerization can be established by suitable combinations of these upper limits and lower limits. The degree of polymerization of the present oxidized cellulose is more preferably 60 to 600, still more preferably 70 to 600, even more preferably 80 to 600, still more preferably 80 to 550, still more preferably 80 to 500, still more preferably 80 to 450, and particularly preferably 80 to 400.

The degree of polymerization of the oxidized cellulose can be adjusted by changing, e.g., the following during the oxidation reaction: the reaction time, reaction temperature, pH, and available chlorine concentration from the hypochlorous acid or salt thereof. In specific terms, since the degree of polymerization exhibits a declining trend as the degree of oxidation increases, for example, increasing the oxidation reaction time and/or reaction temperature is an example of methods for reducing the degree of polymerization. In another method, the degree of polymerization of the oxidized cellulose can be adjusted using the stirring conditions in the reaction system during the oxidation reaction. For example, under conditions in which the reaction system is made satisfactorily uniform, using, e.g., stirring blades, the oxidation reaction proceeds smoothly and a trend is assumed of a declining degree of polymerization. On the other hand, under conditions that facilitate the appearance of inadequate stirring of the reaction system, such as stirring with a stirrer, the reaction readily becomes nonuniform and it is difficult to satisfactorily reduce the degree of polymerization of the present cellulose fiber. The degree of polymerization of the oxidized cellulose also tends to be changed depending on the selection of the starting cellulose. Due to this, the degree of polymerization of the oxidized cellulose can also be adjusted through selection of the cellulose raw material. In this Description, the degree of polymerization of the oxidized cellulose is the average degree of polymerization measured by a viscosity method (viscosity-average degree of polymerization). The details are in accordance with the method described in the examples below.

[The carboxy group content]

The carboxy group content in the present oxidized cellulose is preferably 0.30 to 2.0 mmol/g. A satisfactory ease-of-fibrillatability can be imparted to the oxidized cellulose when this carboxy group content is at least 0.30 mmol/g. This makes it possible, even when the fibrillation treatment is carried out under mild conditions, to obtain a nanocellulose-containing slurry of uniform quality and to improve the viscosity stability, handling characteristics, and coating characteristics of the slurry. When, on the other hand, the carboxy group content is not more than 2.0 mmol/g, excessive decomposition of the cellulose during the fibrillation treatment can be inhibited and a nanocellulose of uniform quality, having a low ratio of particulate cellulose, can be obtained. In view of these considerations, the carboxy group content in the present oxidized cellulose is more preferably at least 0.35 mmol/g, still more preferably at least 0.40 mmol/g, even more preferably at least 0.42 mmol/g, still more preferably at least 0.50 mmol/g, still more preferably in excess of 0.50 mmol/g, and still more preferably at least 0.55 mmol/g. The upper limit on the carboxy group content is more preferably not more than 1.5 mmol/g, still more preferably 1.2 mmol/g, even more preferably not more than 1.0 mmol/g, and still more preferably 0.9 mmol/g. Preferred ranges for the carboxy group content can be established by suitable combinations of these upper limits and lower limits. The carboxy group content of the present oxidized cellulose is more preferably 0.35 to 2.0 mmol/g, still more preferably 0.35 to 1.5 mmol/g, even more preferably 0.40 to 1.5 mmol/g, still more preferably 0.50 to 1.2 mmol/g, still more preferably from more than 0.50 to 1.2 mmol/g, and still more preferably 0.55 to 1.0 mmol/g.

With regard to the determination of the carboxy group content (mmol/g) in the oxidized cellulose, a 0.1 M aqueous hydrochloric acid solution is added to an aqueous solution of the oxidized cellulose mixed with water, to bring the pH to 2.5; a 0.05 N aqueous sodium hydroxide solution is then added dropwise and the electrical conductivity is measured until the pH reaches 11.0; and the carboxy group content is the value calculated using the following formula from the amount (a) of sodium hydroxide consumed in the weak acid neutralization stage where the electrical conductivity undergoes a gentle change. The details are in accordance with the method described in the examples below. The carboxy group content in the oxidized cellulose can be adjusted by changing, e.g., the reaction time, reaction temperature, pH of the reaction solution, and so forth, during the oxidation reaction.

$$\text{carboxy group content} = a \text{ (mL)} \times 0.05 / \text{ mass (g) of the oxidized cellulose}$$

The present oxidized cellulose can be obtained, for example, by subjecting a cellulose raw material to oxidation under conditions that provide a relatively high concentration (for example, 14 mass% to 43 mass%) in the reaction system for the available chlorine concentration from the hypochlorous acid or salt thereof.

The present oxidized cellulose advantageously has a structure in which at least two of the hydroxyl groups in the glucopyranose ring constituting cellulose have been oxidized, and more specifically has a structure in which the carboxy group has been introduced by the oxidation of the hydroxyl groups at positions 2 and 3 of the glucopyranose ring. Preferably the hydroxyl group at position 6 in the glucopyranose ring is not oxidized in the present oxidized cellulose and remains a hydroxyl group. The positions of the carboxy groups in the glucopyranose rings present in the oxidized cellulose can be analyzed using solid-state $^{13}$C-NMR spectroscopy.

The presence of an oxidized structure can be confirmed by observation in this solid-state $^{13}$C-NMR spectrum of a peak corresponding to the carboxy groups at positions 2 and 3 in the glucopyranose ring. Here, the peak corresponding to the carboxy groups at positions 2 and 3 can be observed as a broad peak in the range of 165 ppm to 185 ppm. The broad peak referenced here can be assessed using the peak area ratio.

That is, a baseline is drawn for the peak in the 165 ppm to 185 ppm range in the NMR spectrum; the overall area value is determined; the ratio of the two peak area values (larger area value/smaller area value) - obtained by the perpendicular partitioning of the area value at the peak top - is then determined; and the conclusion is drawn that this is a broad peak when this peak area value ratio is at least 1.2.

The presence/absence of this broad peak can also be evaluated using the ratio between the length L of the baseline for the range of 165 ppm to 185 ppm and the length L' of the perpendicular line from the peak top to the baseline. Thus, it can be concluded that the broad peak is present when the ratio L'/L is greater than or equal to 0.1. This ratio L7L may be greater than or equal to 0.2, greater than or equal to 0.3, greater than or equal to 0.4, or greater than or equal to 0.5. While the upper limit for the ratio L7L is not particularly limited, in general it should be less than or equal to 3.0 and may be less than or equal to 2.0 or less than or equal to 1.0.

The structure of the aforementioned glucopyranose ring in the present nanocellulose can also be determined by analysis based on the methodology described in Sustainable Chem. Eng. 2020, 8, 48, 17800-17806.

The oxidized cellulose according to the present disclosure has an excellent fibrillatability. In particular, even when the fibrillation treatment is carried out using mild conditions, the present oxidized cellulose can be uniformly microfine-sized and thus exhibits an excellent ease-of-fibrillatability. In addition, when the microfine-sized oxidized cellulose is converted into a slurry state by mixing with inorganic particles, e.g., a pigment, the slurry viscosity is stable with elapsed time and the handling characteristics and coating characteristics are also excellent. Moreover, because N-oxyl compounds are not present, the impact on, for example, the environment, can be reduced.

[Method for producing the oxidized cellulose]

The method for producing the present oxidized cellulose is described in the following. The present oxidized cellulose can be produced by a method comprising a step of oxidizing a cellulose raw material with a hypochlorous acid or a salt thereof.

The cellulose raw material should be a material that is mainly cellulose, but is not otherwise particularly limited, and can be exemplified by pulp, natural cellulose, regenerated cellulose, and microfine celluloses provided by depolymerizing a cellulose by mechanical treatment. A commercial product, e.g., crystalline cellulose sourced from pulp, can be used as such as the cellulose raw material. Otherwise, unused biomass containing large amounts of a cellulose component, e.g., soy pulp or soybean skin, may be used as a raw material. The cellulose raw material may be pretreated with an alkali at a suitable concentration with the goal of facilitating permeation of the oxidant used into the starting pulp.

The hypochlorous acid or salt thereof used to oxidize the cellulose raw material can be exemplified by an aqueous hypochlorous acid, sodium hypochlorite, potassium hypochlorite, calcium hypochlorite, and ammonium hypochlorite. Among these, sodium hypochlorite is preferred from the standpoint of ease of handling.

Methods for producing the present oxidized cellulose by oxidation of a cellulose raw material can be exemplified by a method in which the cellulose raw material is mixed with a reaction solution containing a hypochlorous acid or a salt thereof. Water is preferably contained as the solvent based on considerations of easy handling and impeding the production of secondary reactions. The available chlorine concentration in the reaction solution of the hypochlorous acid or salt thereof is preferably 6 to 43 mass%, more preferably 7 to 43 mass%, still more preferably 10 to 43 mass%, and even more preferably 14 to 43 mass%. When the available chlorine concentration in the reaction solution is in the indicated range, the carboxy group content in the oxidized cellulose can then be satisfactorily high and fibrillation of the oxidized cellulose can be easily performed when nanocellulose production is carried out.

From the standpoint of efficiently providing a satisfactorily high carboxy group content in the oxidized cellulose, the available chlorine concentration in the reaction solution is more preferably at least 15 mass%, still more preferably at least 18 mass%, and even more preferably at least 20 mass%. Moreover, based on a consideration of inhibiting excessive decomposition of the cellulose during fibrillation, the available chlorine concentration of the reaction solution is more preferably not more than 40 mass% and still more preferably not more than 38 mass%. Ranges for the available chlorine concentration of the reaction solution can be established by suitable combinations of these upper limits and lower limits. The range for this available chlorine concentration is more preferably 16 to 43 mass% and still more preferably 18 to 40 mass%.

The available chlorine concentration of a hypochlorous acid or salt thereof is defined as follows. The hypochlorous acid is a weak acid occurring as the aqueous solution, and hypochlorites are compounds in which the hydrogen of the hypochlorous acid has been converted into another cation. For example, since sodium hypochlorite, which is a salt of the hypochlorous acid, exists in solvent (preferably in an aqueous solution), the concentration is measured as the available amount of chlorine in solution rather than the sodium hypochlorite concentration. With respect here to the available chlorine from sodium hypochlorite, the oxidizing power of the divalent oxygen atom generated by the decomposition of sodium hypochlorite corresponds to 2 atom-equivalents of monovalent chlorine, and thus the bound chlorine atom of sodium hypochlorite (NaClO) has the same oxidizing power as two atoms of nonbound chlorine ($Cl_2$) and the available chlorine = 2 × (chlorine in NaClO). In a specific measurement means, the available chlorine concentration is measured by precisely weighing the sample; adding water, potassium iodide, and acetic acid; allowing the mixture to stand; and titrating the liberated iodine with a sodium thiosulfate solution using an aqueous starch solution as indicator.

The oxidation reaction of the cellulose raw material by a hypochlorous acid or a salt thereof should be carried out while adjusting the pH into the range from 5.0 to 14.0. Within this range, the oxidation reaction of the cellulose raw material can be satisfactorily developed and the amount of carboxy group in the oxidized cellulose can be brought to satisfactorily high levels. This in turn makes it possible to readily carry out fibrillation of the oxidized cellulose. The pH of the reaction system is more preferably greater than or equal to 6.0, still more preferably greater than or equal to 7.0, and even more preferably greater than or equal to 8.0. The upper limit on the pH of the reaction system is more preferably less than or equal to 13.5 and still more preferably less than or equal to 13.0. The pH range for the reaction system is more preferably 7.0 to 14.0 and still more preferably 8.0 to 13.5.

The method for producing the present oxidized cellulose is further described in the following for the example of the use of a hypochlorous acid or sodium hypochlorite as the salt thereof.

When oxidation of the cellulose raw material is carried out using sodium hypochlorite, the reaction solution is then preferably an aqueous sodium hypochlorite solution. The procedure for adjusting the available chlorine concentration of the aqueous sodium hypochlorite solution to the target concentration (for example, a target concentration of 6 mass% to 43 mass%) can be exemplified by the following: concentration of an aqueous sodium hypochlorite solution that has a lower available chlorine concentration than the target concentration; dilution of an aqueous sodium hypochlorite solution that has a higher available chlorine concentration than the target concentration; and dissolution of crystalline sodium hypochlorite (for example, sodium hypochlorite pentahydrate) in solvent. Among these, adjustment to the available chlorine concentration serving as the oxidant by dilution of an aqueous sodium hypochlorite solution or dissolution of crystalline sodium hypochlorite in solvent is preferred because there is then little autodecomposition (i.e., little decline in the available chlorine concentration) and because the available chlorine concentration may be easily adjusted.

There are no particular limitations on the method for mixing the cellulose raw material with the aqueous sodium hypochlorite solution, but, viewed from the perspective of ease of operation, mixing is preferably performed by adding the cellulose raw material to the aqueous sodium hypochlorite solution.

In order to efficiently develop the oxidation reaction of the cellulose raw material, the cellulose raw material + aqueous sodium hypochlorite solution mixture is preferably stirred during the oxidation reaction. The stirring method can be exemplified by the use of a magnetic stirrer, stir bar, stirring blade-equipped stirrer (ThreeOne Motor), homomixer, dispersing-type mixer, homogenizer, external circulation mixer, and so forth. Among these, and based on considerations of smoothly carrying out the oxidation reaction of the cellulose raw material and ease of adjusting the degree of polymerization of the oxidized cellulose to or below a prescribed value, a method using one or two or more selections from shear-based stirrers, e.g., homomixers, homogenizers, and so forth, stirring blade-equipped stirrers, and dispersing-type mixers is preferred, while a method using a stirring blade-equipped stirrer is particularly preferred. When a stirring blade-equipped stirrer is used, a device provided with known stirring blades, e.g., propeller blades, paddle blades, turbine blades, and so forth, can be used as the stirrer. When a stirring blade-equipped stirrer is used, stirring is preferably performed at a rotation rate of 50 to 300 rpm.

The reaction temperature in the oxidation reaction is preferably 15°C to 100°C and is more preferably 20°C to 90°C. The pH in the reaction system declines during the reaction accompanying the production of the carboxy group in the cellulose raw material due to the oxidation reaction. Due to this, and from the standpoint of efficiently carrying out the oxidation reaction, the oxidation reaction is preferably carried out while adjusting the pH in the reaction system by adding a base (for example, sodium hydroxide) or an acid (for example, hydrochloric acid) to the reaction system. The reaction time for the oxidation reaction can be established in accordance with the degree to which the oxidation is to be advanced; however, about 15 minutes to 50 hours is preferred. When the pH in the reaction system is made 10 or more, preferably the reaction temperature is made 30°C or above and/or the reaction time is made 30 minutes or more.

An oxidized cellulose, comprising an oxide of the cellulose raw material by a hypochlorous acid or a salt thereof, can be obtained by subjecting the solution containing the oxidized cellulose yielded by the aforementioned reaction, to a known separation process, e.g., filtration, and as necessary to additional purification. In addition, based on considerations of improving the filtration behavior and yield in the separation process, at least a portion of the salt form ($-COO^- X^+$ : $X^+$ indicates a cation, e.g., sodium, lithium) of the carboxy groups produced by oxidation can be converted into the proton form ($-COO^- H^+$) by the addition, prior to the separation process, e.g., filtration, of an acid to the oxidized cellulose-containing solution, for example, to bring the pH to 4.0 or below.

In the infrared absorption spectrum, the proton form presents a peak in the vicinity of 1720 cm$^{-1}$ and the salt form presents a peak in the vicinity of 1600 cm$^{-1}$, which enables these to be distinguished.

The oxidized cellulose-containing solution yielded by the aforementioned reaction may be supplied as such to the fibrillation treatment.

When the pH of the oxidized cellulose-containing solution has been brought to 4.0 or below for the separation process, in order to improve the handling characteristics when provided to the ensuing fibrillation treatment, at least a portion of the carboxy groups can be converted to the salt form ($-COO^- X^+$ : $X^+$ indicates a cation, e.g., sodium, lithium), for example, by bringing the pH to 6.0 or higher by the addition of a base. In addition, the oxidized cellulose-containing solution may be converted into an oxidized cellulose-containing composition, for example, by replacing the solvent in the former. At least a portion of the carboxy groups can also be converted into the salt form ($-COO^- X^+$ : $X^+$ indicates a cation, e.g., sodium, lithium) in the oxidized cellulose-containing composition, for example, by establishing alkaline conditions of 10 or higher for the pH.

In order to control the properties of the oxidized cellulose, the method for producing the present oxidized cellulose may additionally comprise a step of mixing the obtained oxidized cellulose with a modifying group-bearing compound. The modifying group-bearing compound should be a compound that has a modifying group that can form an ionic bond or covalent bond with the carboxy groups or hydroxyl groups possessed by the oxidized cellulose, but is not otherwise particularly limited. Compounds having a modifying group that can form an ionic bond can be exemplified by primary amines, secondary amines, tertiary amines, quaternary ammonium compounds, and phosphonium compounds. Compounds having a modifying group that can form a covalent bond can be exemplified by alcohols, isocyanate compounds, and epoxy compounds.

In accordance with the preceding, the present oxidized cellulose encompasses the embodiments represented by the salt forms, proton forms, and modified forms as provided by a modifying group. The nanocellulose obtained from the present oxidized cellulose also encompasses the embodiments represented by the salt forms, proton forms, and modified forms as provided by a modifying group.

The present oxidized cellulose may also take the form of a mixture with a dispersion medium. That is, an aspect of the present invention is an oxidized cellulose dispersion in which the present oxidized cellulose dispersed in a dispersion medium. This dispersion medium can be exemplified by the same dispersion media as described below.

«The nanocellulose»

The nanocellulose according to the present disclosure (also referred to as the "present nanocellulose" in the following) can be obtained by carrying out fibrillation and nanosizing on the oxidized cellulose yielded by oxidation of a cellulose raw material. That is, the present nanocellulose can be produced by a method comprising a step of oxidizing a cellulose raw material using a hypochlorous acid or a salt thereof, and a step of fibrillating the oxidized cellulose yielded by the preceding step. The oxidation step is as has been described above.

"Nanocellulose" is also referred to as "microfine cellulose fiber" because it is a fibrous cellulose provided by the microfine-sizing of oxidized fibrous cellulose.

The method for fibrillating the oxidized cellulose can be exemplified by methods that employ mechanical fibrillation. Here, nanocellulose (also referred to as "nanocellulose" in the following) is a general term for the products provided by the nanosizing of cellulose and encompasses, e.g., cellulose nanofiber, cellulose nanocrystals, and so forth.

The method for carrying out mechanical fibrillation can be exemplified by methods that use various mixers or stirring devices, such as, for example, screw mixers, paddle mixers, dispersing mixers, turbine mixers, high-speed homomixers, high-pressure homogenizers, ultrahigh-pressure homogenizers, double cylinder homogenizers, ultrasonic homogenizers, water-current counter-collision dispersers, beaters, disk refiners, conical refiners, double disk refiners, grinders, single-screw kneaders, multi-screw kneaders, planetary centrifugal mixers, and vibrating agitators. A single one of these devices may be used by itself or a combination of two or more of these devices may be used, and preferably nanocellulose production is carried out by nanosizing the oxidized cellulose by treating the oxidized cellulose in a dispersion medium.

From the standpoint of being able to efficiently produce nanocellulose in which fibrillation is more advanced, the present oxidized cellulose may be fibrillated using, for example, a method that employs an ultrahigh-pressure homogenizer. When fibrillation is carried out using an ultrahigh-pressure homogenizer, the pressure during the fibrillation treatment is preferably at least 100 MPa, more preferably at least 120 MPa, and still more preferably at least 150 MPa. The number of times the fibrillation treatment is carried out is not particularly limited, but, from the standpoint of achieving a satisfactory development of the fibrillation, the fibrillation treatment is preferably carried out at least two times and more preferably at least three times.

The present oxidized cellulose exhibits an excellent ease-of-fibrillatability, and as a consequence can be satisfactorily fibrillated even when mild stirring is applied for the fibrillation method, as with, for example, a planetary centrifugal mixer or vibrating agitator, and the present oxidized cellulose is thus favorable from the standpoint of enabling the acquisition of a homogenized nanocellulose.

A planetary centrifugal mixer is a device that mixes a material within a container by rotating and revolving the material-loaded container. A planetary centrifugal mixer can realize a milder stirring because it carries out stirring without using stirring blades. The revolution rate and rotation rate during stirring with a planetary centrifugal mixer can be established as appropriate, and, for example, the revolution rate can be 400 to 3,000 rpm and the rotation rate can be 200 to 1,500 rpm. When a planetary centrifugal mixer is used, and viewed from the standpoints of securing a uniform quality while implementing mild stirring, the fibrillation treatment is preferably carried out using the following conditions: stirring at a revolution rate of 1,200 to 2,500 rpm and a rotation rate of 600 to 1,000 rpm for 3 to 15 minutes. The revolution rate is more preferably 1,500 to 2,300 rpm and the rotation rate is more preferably 700 to 950 rpm. When fibrillation of the present oxidized cellulose is carried out using a planetary centrifugal mixer, the concentration of the aqueous oxidized cellulose dispersion functioning as the material may be adjusted as appropriate, but, for example, is 0.01 to 1.0 mass% and preferably 0.1 to 0.5 mass%.

A vortex mixer (touch mixer) is an example of a vibrating agitator. In a vortex mixer, stirring is carried out by the formation of a vortex in a liquid material in a container. Since stirring is carried out without using stirring blades when a vibrating agitator, e.g., a vortex mixer, is used, a milder stirring or mixing can be realized. Since mild stirring can be realized using simple equipment when a vibrating agitator, e.g., a vortex mixer, is used, a vibrating agitator such as a vortex mixer is excellent from the standpoints of the production equipment and production cost. The fibrillation treatment is preferably carried out using the following conditions: a rotation rate for the vortex mixer of, for example, 600 to 3,000 rpm and stirring for 3 to 15 minutes. When the present oxidized cellulose is fibrillated using a vortex mixer, the concentration of the aqueous oxidized cellulose dispersion functioning as the material may be adjusted as appropriate, but is, for example, 0.01 to 1.0 mass% and preferably 0.1 to 0.5 mass%.

The fibrillation treatment is preferably carried out in a state in which the present oxidized cellulose is mixed with a dispersion medium. This dispersion medium is not particularly limited and can be selected as appropriate in accordance with the objectives. Specific examples of the dispersion medium are water, alcohols, ethers, ketones, N,N-dimethylformamide, N,N-dimethylacetamide, and dimethyl sulfoxide. A single one of these may be used by itself as the solvent or two or more may be used in combination.

Of the dispersion media referenced above, the alcohols can be exemplified by methanol, ethanol, isopropanol, isobutanol, sec-butyl alcohol, tert-butyl alcohol, methyl cellosolve, ethylene glycol, and glycerol. The ethers can be exemplified by ethylene glycol dimethyl ether, 1,4-dioxane, and tetrahydrofuran. The ketones can be exemplified by acetone and methyl ethyl ketone.

Isolation of the oxidized cellulose, and of the nanocellulose yielded by its fibrillation, is facilitated by the use of an organic solvent as the dispersion medium in the fibrillation treatment. Since nanocellulose dispersed in organic solvent is then obtained, mixing with, e.g., resin soluble in the organic solvent or monomer that is a starting material for such a resin, is facilitated. The nanocellulose dispersion of nanocellulose yielded by fibrillation dispersed in a dispersion medium of water and/or organic solvent, can be used for mixing with various components, e.g., resins, rubbers, solid particles, and so forth.

[The average fiber width]

The average fiber width of the present nanocellulose is preferably 1 to 200 nm. In particular, the present oxidized cellulose is favorable from the standpoint of making it possible to obtain nanocellulose that has been satisfactorily nanosized by the fibrillation treatment to an average fiber width of 1 to 20 nm, preferably 1 to 10 nm, and more preferably 1 to 5 nm. In addition, when the average fiber width of the present nanocellulose is satisfactorily small at 1 to 5 nm, a slurry containing this nanocellulose has a stabilized viscosity and exhibits excellent handling characteristics and coating characteristics. The average fiber width of the present nanocellulose is more preferably not more than 4.8 nm, still more preferably not more than 4.5 nm, and even more preferably not more than 4.2 nm. There are no particular limitations on the lower limit for the average fiber width from the perspective of ease-of-fibrillatability. However, when the average fiber width is less than 1 nm, this approaches a cellulose single molecule state and the quality as nanocellulose readily becomes nonuniform. As a consequence, the average fiber width is preferably at least 1 nm and more preferably at least 1.5 nm.

[The average fiber length]

The average fiber length of the present nanocellulose is preferably 100 to 2,000 nm. The average fiber length is more preferably 100 to 1,000 nm, still more preferably 100 to 500 nm, and even more preferably 100 to 400 nm.

[The aspect ratio]

The present nanocellulose preferably has an aspect ratio (average fiber length/average fiber width), which represents the ratio of the average fiber length to the average fiber width, of 20 to 200. This aspect ratio is more preferably at least 30 and still more preferably at least 40. In addition, the aspect ratio is more preferably not more than 180 and still more preferably not more than 150.

The average fiber width and average fiber length are the values calculated as follows: water is mixed with the nanocellulose so as to provide a nanocellulose concentration of about 1 to 10 ppm; the satisfactorily diluted aqueous cellulose dispersion is naturally dried on a mica substrate; observation of the shape of the nanocellulose is then carried out using a scanning probe microscope; some number of fibers are randomly selected from the resulting image; and the cross-sectional height of the shape image = fiber width and the length of the perimeter $\div$ 2 = fiber length. Image processing software may be used for this calculation of the average fiber width and average fiber length. When this is done, the image processing conditions may be freely selected; however, there will be instances in which, even for the same image, differences in the calculated values may be produced depending on the image processing conditions. The range of difference in the values due to the image processing conditions is preferably in the $\pm$ 100 nm range for the average fiber length. The range of difference in the values due to the conditions is preferably in the $\pm$ 10 nm range for the average fiber width. More specifically, the measurement method follows the method described in the examples below.

[The light transmittance]

The present oxidized cellulose can be thoroughly fibrillated even using mild fibrillation conditions to yield nanocellulose having a satisfactorily small fiber width. In addition, because the resulting nanocellulose has a satisfactorily small fiber width, when dispersed in a dispersion medium the cellulose fiber causes little light scattering, inter alia, and exhibits a high light transmittance. Accordingly, the present nanocellulose can be effectively utilized in those applications where transparency is required.

Specifically, preferably a light transmittance value of at least 60% is exhibited by the aqueous nanocellulose dispersion obtained by carrying out a fibrillation treatment on an aqueous dispersion of the present oxidized cellulose having an oxidized cellulose concentration of 0.1 mass%, using a planetary centrifugal mixer and conditions of 10 minutes, a revolution rate of 2,000 rpm, and a rotation rate of 800 rpm. The light transmittance of this aqueous nanocellulose dispersion is more preferably at least 70%, still more preferably at least 75%, and even more preferably at least 80%. This light transmittance is the value measured at a wavelength of 660 nm using a spectrophotometer.

In addition, preferably a light transmittance value of at least 60% is exhibited by the aqueous nanocellulose dispersion obtained by carrying out a fibrillation treatment on an aqueous dispersion of the present oxidized cellulose having an oxidized cellulose concentration of 0.1 mass%, using a vortex mixer and conditions of 10 minutes and a rotation rate of 3,000 rpm. The light transmittance of this aqueous nanocellulose dispersion is more preferably at least 70%, still more preferably at least 75%, and even more preferably at least 80%.

The reasons why the oxidized cellulose according to the present disclosure exhibits an excellent fibrillatability (particularly ease-of-fibrillatability) and provides a high quality slurry are not clear, but the following is generally hypothesized. Fibrillation advances via the scission of the hydrogen bonds between the cellulose microfibrils. During the oxidation treatment using a hypochlorous acid or a salt thereof, a reduction in the degree of polymerization of the microfibrils (i.e., a shortening of the cellulose molecular chains) occurs accompanying the progression of oxidation. In the case of oxidation with a hypochlorous acid or a salt thereof at a relatively high concentration, this decline in the degree of polymerization progresses more readily accompanying the increase in the degree of oxidation, than is the case, for example, for TEMPO oxidation. As a consequence, it is thought that the fibrillatability of the oxidized cellulose is enhanced in the present embodiment because the oxidation treatment reduces the number of hydrogen bonds in each microfibril to be cleaved by fibrillation and because repulsion between microfibrils is strengthened due to an increase in the carboxy group content accompanying the progress of the oxidation. In addition, it is thought that the improved fibrillatability of the oxidized cellulose enables the production of a nanocellulose that can exhibit an enhanced viscosity stability, handling characteristics, and coating characteristics for the slurry.

[Other embodiments]

An oxidized cellulose of a cellulose raw material oxidized by an oxidant is provided in another advantageous embodiment of the present disclosure. This oxidized cellulose can also be referred to as an oxidized cellulose that is an oxide of a cellulose raw material. A light transmittance of at least 60% is exhibited by the aqueous nanocellulose dispersion obtained by carrying out a fibrillation treatment on an aqueous dispersion of the oxidized cellulose according to this embodiment having an oxidized cellulose concentration of 0.1 mass%, using a planetary centrifugal mixer and conditions of 10 minutes, a revolution rate of 2,000 rpm, and a rotation rate of 800 rpm.

In addition, in another advantageous embodiment of the present disclosure, a light transmittance of at least 60% is exhibited by the aqueous nanocellulose dispersion obtained by carrying out a fibrillation treatment on an aqueous dispersion of the present oxidized cellulose having an oxidized cellulose concentration of 0.1 mass%, using a vortex mixer and conditions of 10 minutes and a rotation rate of 3,000 rpm.

**That** is, with the oxidized celluloses according to these other embodiments, fibrillation proceeds thoroughly even under mild fibrillation conditions and a nanocellulose having a satisfactorily small fiber width of about 1 to 5 nm can be obtained. Because the resulting nanocellulose has a satisfactorily small fiber width, when dispersed in a dispersion medium the cellulose fiber causes little light scattering, inter alia, and exhibits a high light transmittance. This enables and supports effective utilization in those applications where transparency is required. In addition, nanocellulose provided by fibrillation of oxidized cellulose according to these other embodiments, even when the fibrillation treatment has been carried out under mild conditions, can provide a stabilization of the slurry viscosity when made into a nanocellulose-containing slurry, and in addition can provide good handling characteristics and good coating characteristics for the slurry.

The aforementioned oxidant can be exemplified by halogen, a hypochlorous acid or a salt thereof, and peroxides. Among these, the hypochlorous acid and its salts are preferred from the standpoints of being able to obtain a homogeneous nanocellulose, having a low environmental impact, and being inexpensive. The hypochlorous acid and its salts are as already described above.

The hereinabove described nanocellulose and nanocellulose dispersions containing same can be used in a variety of applications. In specific terms, for example, they may be used as a reinforcing material when mixed with various materials (e.g., resins, fibers, rubbers, and so forth) and may be used as a thickener or dispersant in various applications (e.g., foods, cosmetics, medical products, paints, inks, and so forth). The nanocellulose dispersion may also be formed into a film and used as various sheets and films. The fields of application of the present nanocellulose and nanocellulose dispersions containing same are not particularly limited, and they may be used in the production of products in various fields, e.g., automotive components, machine parts, electrical appliances, electronic devices, cosmetics, medical products, building materials, daily necessities, stationery, and so forth. Moreover, for example, the use of the nanocellulose or nanocellulose dispersion containing same as an additive to a slurry containing inorganic particles, such as a pigment, is advantageous from the standpoint of enabling improvements in the viscosity stability, handling characteristics, and coating performance of the slurry.

Examples

The present invention is specifically described in the following using examples, but the present invention is not limited to or by these examples. Unless specifically indicated otherwise, in the following "parts" indicates "mass parts" and "%" indicates "mass%".

(1) Production of oxidized celluloses and nanocelluloses

[Production Example 1]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

350 g of sodium hypochlorite pentahydrate crystals, which had an available chlorine concentration of 42 mass%, was introduced into a beaker and pure water was added and stirring was performed to obtain an aqueous sodium hypochlorite solution having an available chlorine concentration of 21 mass%. To this was added 35 mass% hydrochloric acid with stirring to provide an aqueous solution having a pH of 11.0. This aqueous sodium hypochlorite solution was heated to 30°C with a thermostatted water bath while stirring at 200 rpm using a stirrer from Shinto Scientific Co., Ltd. (ThreeOne Motor, BL600) and using a propeller stirring blade; this was followed by the addition of 50 g of the aforementioned mechanically fibrillated softwood kraft pulp (carboxy group content: 0.05 mmol/g).

After the introduction of the cellulose raw material, and while holding the temperature at 30°C on the same thermostatted water bath, an oxidation reaction was run for 30 minutes while stirring at 200 rpm using a propeller stirring blade and the aforementioned stirrer while adjusting the pH during the reaction to 11.0 by the addition of 48 mass% sodium hydroxide. After the completion of the reaction, solid-liquid separation was carried out on the product by suction filtration using a PTFE membrane filter having an aperture of 0.1 μm to obtain an oxidized cellulose A. The obtained oxidized cellulose A was washed with pure water, and the carboxy group content measured post-washing for the residue (oxidized cellulose A) was 0.45 mmol/g.

A 0.1% dispersion was then prepared by the addition of pure water to the oxidized cellulose A, and an aqueous CNF dispersion A, i.e., a nanocellulose dispersion, was obtained by treatment for 10 minutes using a "THINKYMIXER ARE-310" planetary centrifugal mixer from the THINKY Corporation and conditions of a revolution rate of 2,000 rpm and a rotation rate of 800 rpm in mix mode.

In addition, the N-oxyl compound-derived nitrogen component in the oxidized cellulose A was measured as the amount of nitrogen using a total trace nitrogen analyzer (instrument name: TN-2100H, from Mitsubishi Chemical Analytech Co., Ltd.); the increment from the starting pulp was calculated, giving a result of not more than 1 ppm.

The available chlorine concentration in the aqueous sodium hypochlorite solution was measured using the following method.

(Measurement of the available chlorine concentration in the aqueous sodium hypochlorite solution)

0.582 g of an aqueous solution of sodium hypochlorite pentahydrate crystals added to pure water was precisely weighed out, and 50 mL of pure water was added and 2 g of potassium iodide and 10 mL of acetic acid were added, followed immediately by tight sealing and standing for 15 minutes in a dark location. After the standing for 15 minutes, the liberated iodine was titrated with a 0.1 mol/L sodium thiosulfate solution, which resulted in a titration volume of 34.55 mL (indicator: starch reagent solution). Correction was performed by carrying out a separate blank test. Since 1 mL of the 0.1 mol/L sodium thiosulfate solution corresponded to 3.545 mg Cl, the available chlorine concentration in the aqueous sodium hypochlorite solution was 21 mass%.

The carboxy group content in the oxidized cellulose was measured using the following method.

(Measurement of the carboxy group content)

To 60 mL of an aqueous oxidized cellulose dispersion adjusted to an oxidized cellulose concentration of 0.5 mass%, was added 0.1 M aqueous hydrochloric acid solution to bring the pH to 2.5. This was followed by the dropwise addition of a 0.05 N aqueous sodium hydroxide solution and measurement of the electrical conductivity until the pH reached 11.0, and the carboxy group content (mmol/g) was calculated using the following formula from the amount (a) of sodium hydroxide consumed in the weak acid neutralization stage where the electrical conductivity undergoes a gentle change.

$$\text{carboxy group content} = a \text{ (mL)} \times 0.05 / \text{ mass (g) of the oxidized cellulose}$$

[Production Example 2]

An oxidized cellulose B and an aqueous CNF dispersion B were obtained by carrying out treatment using the same conditions as in Production Example 1, but using 120 minutes for the reaction time in the oxidation reaction.

[Production Example 3]

An oxidized cellulose C and an aqueous CNF dispersion C were obtained by carrying out treatment using the same conditions as in Production Example 1, but using 120 minutes for the reaction time in the oxidation reaction and changing the cellulose raw material to a powdered cellulose (VP-1) from the TDI Corporation.

[Production Example 4]

An oxidized cellulose D and an aqueous CNF dispersion D were obtained by carrying out treatment using the same conditions as in Production Example 1, but using 120 minutes for the oxidation reaction time and changing the cellulose raw material to a powdered cellulose (KC Flock W-100GK) from Nippon Paper Industries Co., Ltd.

[Production Example 5]

An oxidized cellulose E and an aqueous CNF dispersion E were obtained by carrying out treatment using the same conditions as in Production Example 1, but using 240 minutes for the oxidation reaction time.

[Production Example 6]

An oxidized cellulose F and an aqueous CNF dispersion F were obtained by carrying out treatment using the same conditions as in Production Example 1, but using 360 minutes for the oxidation reaction time.

[Production Example 7]

An oxidized cellulose G and an aqueous CNF dispersion G were obtained by carrying out treatment using the same conditions as in Production Example 1, but using 50°C for the oxidation reaction temperature.

[Production Example 8]

An oxidized cellulose H and an aqueous CNF dispersion H were obtained by carrying out treatment using the same conditions as in Production Example 1, but using 480 minutes for the oxidation reaction time.
A sample was prepared by freeze-frying the oxidized cellulose provided by the particular production example and then holding for at least 24 hours at 23°C and 50% RH, and the solid-state $^{13}$C-NMR of the sample was measured. As a result, in all instances it was confirmed that a structure was present in which the hydroxyl groups at positions 2 and 3 of the glucopyranose ring were oxidized with the introduction of carboxy groups. The solid-state $^{13}$C-NMR measurement conditions are given below.

(1) sample tube: zirconia tube (4 mm diameter)

(2) magnetic field strength: 9.4 T (1H resonance frequency: 400 MHz)

(3) MAS rotation rate: 15 kHz

(4) pulse sequence: CPMAS method

(5) contact time: 3 ms

(6) waiting time: 5 s

(7) number of scans: 10,000 to 15,000

(8) measurement instrument: JNM ECA-400 (JEOL Ltd.)

The results obtained using a model molecule for said oxidized cellulose as the sample and carrying out measurement using two-dimensional NMR also confirmed that the oxidized cellulose yielded by each production example had a structure in which the hydroxyl groups at positions 2 and 3 of the glucopyranose ring were oxidized with the introduction of carboxy groups.
With regard to position 6, no change in the spectroscopic data was seen between the solid-state $^{13}$C-NMR of the cellulose raw material and the solid-state $^{13}$C-NMR of the oxidized cellulose, and based on this it was concluded that the hydroxyl group at position 6 was not oxidized and the hydroxyl group remained intact in the oxidized cellulose.

[Comparative Production Example 1]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.
30.0 g of sodium hypochlorite pentahydrate crystals, which had an available chlorine concentration of 43 mass%, was introduced into a 100-mL beaker and pure water and 35 mass% hydrochloric acid were added with stirring to obtain an aqueous solution having an available chlorine concentration of 21 mass% and a pH of 11.0. This aqueous sodium hypochlorite solution was heated to 30°C with a thermostatted water bath while stirring with a stirrer; this was followed by the addition of 0.35 g of the aforementioned mechanically fibrillated softwood kraft pulp.

After the introduction of the cellulose raw material, and while holding the temperature at 30°C on the same thermostatted water bath, stirring with a stirrer was carried out for 30 minutes while adding 48 mass% sodium hydroxide in order to maintain pH 11.0. Solid-liquid separation was then carried out on the product by suction filtration using a PTFE membrane filter having an aperture of 0.1 $\mu$m to obtain an oxidized cellulose P. The obtained residue (oxidized cellulose P) was washed with pure water and the carboxy group content was then measured at 0.42 mmol/g; the amount of the residue was 0.31 g.

A 0.1% dispersion was then prepared by the addition of pure water to the obtained oxidized cellulose P, and an aqueous CNF dispersion P was obtained by carrying out a fibrillation treatment for 10 minutes using a "THINKYMIXER ARE-310" planetary centrifugal mixer from the THINKY Corporation and conditions of a revolution rate of 2,000 rpm and a rotation rate of 800 rpm in mix mode.

[Comparative Production Example 2]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

30.3 g of sodium hypochlorite pentahydrate crystals, which had an available chlorine concentration of 42 mass%, was introduced into a beaker and pure water was added with stirring to bring the available chlorine concentration to 14 mass%. To this was added 35 mass% hydrochloric acid with stirring to obtain an aqueous solution having a pH of 9.0. This aqueous sodium hypochlorite solution was heated to 30°C with a thermostatted water bath while stirring with a stirrer; this was followed by the addition of 0.35 g of the aforementioned mechanically fibrillated softwood kraft pulp.

After the introduction of the cellulose raw material, and while holding the temperature at 30°C on the same thermostatted water bath, an oxidation reaction was run by stirring with a stirrer for 30 minutes while adding 48 mass% sodium hydroxide in order to adjust the pH during the reaction to 9.0. After the completion of the reaction, solid-liquid separation was then carried out on the product by suction filtration using a PTFE mesh filter having an aperture of 0.1 $\mu$m to obtain an oxidized cellulose Q. The obtained 0.12 g residue was washed with pure water. The carboxy group content was measured on the post-wash residue (oxidized cellulose Q) at 1.12 mmol/g.

A 0.1% dispersion was then prepared by the addition of pure water to the oxidized cellulose Q, and an aqueous CNF dispersion Q was obtained by carrying out a fibrillation treatment using the same conditions as in Comparative Production Example 1.

[Comparative Production Example 3]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd. A wet powder was obtained by thoroughly dispersing the mechanically fibrillated cellulose fibers in water and carrying out suction filtration using a PTFE mesh filter having an aperture of 0.1 $\mu$m.

This wet powder (80 mass% moisture fraction, 20 g as the dry powder) was introduced into a container, and 60 L of an ozone · oxygen mixed gas with an ozone concentration of 200 g/m$^3$ was then added and shaking was performed for 2 minutes at 25°C. After standing at quiescence for 6 hours, the ozone and so forth in the container was removed, after which the oxidized cellulose (oxidized cellulose R) was taken out and washed with pure water by suction filtration using a PTFE mesh filter having an aperture of 0.1 $\mu$m. Pure water was added to the obtained oxidized cellulose R to prepare a 2 mass% dispersion, and sodium hydroxide was added to give a 0.3 mass% sodium hydroxide solution. Stirring was carried out for 5 minutes followed by standing at quiescence for 30 minutes at 25°C. Washing with pure water was then performed by suction filtration using a PTFE mesh filter having an aperture of 0.1 $\mu$m. Pure water was added to this oxidized cellulose R to prepare a 0.1% dispersion, and an aqueous CNF dispersion R was obtained by performing a fibrillation treatment using the same conditions as in Comparative Production Example 1.

[Comparative Production Example 4]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

4.92 g sodium periodate was introduced into a beaker and pure water was added to make an aqueous solution (total amount = 600 mL). This aqueous sodium periodate solution was heated to 55°C with a thermostatted water bath while stirring at 200 rpm using a stirrer from Shinto Scientific Co., Ltd. (ThreeOne Motor, BL600) and using a propeller stirring blade; this was followed by the addition of 6 g of the aforementioned mechanically fibrillated softwood kraft pulp.

After the introduction of the cellulose raw material, and while holding the temperature at 55°C on the same thermostatted water bath, stirring was carried out for 3 hours under the same conditions using a stirrer. After the completion of the reaction, solid-liquid separation was carried out on the product by suction filtration using a PTFE membrane filter having an aperture of 0.1 $\mu$m to obtain an oxidized cellulose S, which was then washed with pure water.

The product obtained as described above was then added to a 1 M aqueous acetic acid solution that contained sodium chlorite, and stirring was carried out under the same stirring conditions as above for 48 hours at 25°C. After the completion of the reaction, the product was subjected to solid-liquid separation by suction filtration using a PTFE membrane filter having an aperture of 0.1 $\mu$m, and washing with pure water was performed. Pure water was added to the obtained oxidized cellulose S to prepare a 0.1% dispersion, and an aqueous CNF dispersion S was obtained by performing a fibrillation treatment using the same conditions as in Comparative Production Example 1.

[Comparative Production Example 5]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

0.016 g TEMPO and 0.1 g sodium bromide were introduced into a beaker; pure water was added with stirring to prepare an aqueous solution; and 1.0 g of the aforementioned mechanically fibrillated softwood pulp was added.

This aqueous solution was heated to 25°C on a thermostatted water bath while stirring with a stirrer, after which 0.1 M sodium hydroxide was added with stirring to prepare an aqueous solution having a pH of 10.0. To this was added 2.58 g of an aqueous sodium hypochlorite solution having an available chlorine concentration of 13.2 mass%, and, while holding the temperature at 25°C on the same thermostatted water bath, stirring with a stirrer was performed for 120 minutes while adjusting the pH during the reaction to 10.0 by adding 0.1 M sodium hydroxide.

After the completion of the reaction, the product was subjected to solid-liquid separation by suction filtration using a PTFE membrane filter having an aperture of 0.1 $\mu$m to obtain an oxidized cellulose T. The obtained residue (oxidized cellulose T) was washed with pure water, followed by measurement of the carboxy group content. The carboxy group content was 1.55 mmol/g, and the amount of the residue was approximately 1.0 g. Pure water was added to the obtained oxidized cellulose T to prepare a 0.1% dispersion, and an aqueous CNF dispersion T was obtained by carrying out a fibrillation treatment using the same conditions as in Comparative Production Example 1. The N-oxyl compound-derived nitrogen component in the oxidized cellulose T was measured as the amount of nitrogen using the same conditions as in Production Example 1, and the result, calculated as the increment from the starting pulp, was 5 ppm.

(2) Evaluation 1

[Examples 1-1 to 1-8 and Comparative Examples 1-1 to 1-5]

The following evaluations were carried out using the oxidized celluloses and aqueous CNF dispersions yielded by each of Production Examples 1 to 8 and Comparative Production Examples 1 to 5. The results of the evaluations are given in Table 1.

[Measurement of the viscosity-average degree of polymerization]

The oxidized cellulose was added to an aqueous sodium borohydride solution that had been adjusted to pH 10, and a reduction treatment was run for 5 hours at 25°C. The amount of sodium borohydride was 0.1 g per 1 g of the oxidized cellulose. After the reduction treatment, solid-liquid separation and water washing were performed by suction filtration, and the obtained oxidized cellulose was freeze-dried. 0.04 g of the dried oxidized cellulose was added to 10 mL pure water; stirring was performed for 2 minutes; and 10 mL of a 1 M cupriethylenediamine solution was added and dissolution was carried out. Then, using a capillary viscometer, the efflux time of a blank solution and the efflux time of the cellulose solution were measured at 25°C. The relative viscosity ($\eta_r$), specific viscosity ($\eta_{sp}$), and intrinsic viscosity ($[\eta]$) were determined in order using the following formulas from the efflux time ($t0$) for the blank solution, the efflux time ($t$) for the cellulose solution, and the oxidized cellulose concentration ($c$ [g/mL]), and the degree of polymerization (DP) of the oxidized cellulose was calculated using the viscosity law formula.

$$\eta_r = \eta/\eta_0 = t/t0$$

$$\eta_{sp} = \eta_r - 1$$

$$[\eta] = \eta_{sp}/(100 \times c (1 + 0.28\eta_{sp}))$$

$$DP = 175 \times [\eta]$$

[Measurement of average fiber width]

Pure water was added to each of the aqueous CNF dispersions A to H and P to T obtained as described above in order to adjust the nanocellulose concentration in the aqueous CNF dispersion to 5 ppm. After the concentration adjustment, the aqueous CNF dispersion was naturally dried on a mica substrate, and shape observation of the nanocellulose was performed in AC mode using an "MFP-3D Infinity" from Oxford · Asylum. For the average fiber width, using the software provided with the "MFP-3D Infinity", the number-average fiber width [nm] was determined using cross-sectional height in shape image = fiber width for at least 50 of the fibers.
[Ease-of-fibrillatability]

• Fibrillation method A

Each of the aqueous CNF dispersions A to H and P to T obtained as described above was introduced into a 10 mm-thick quartz cell, and the light transmittance at a wavelength of 660 nm was measured using a spectrophotometer (JASCO V-550). The solids concentration for the aqueous CNF dispersions was 0.1 mass% in all instances. A higher light transmittance indicates a more thorough fibrillation to microfine fibers, even at mild conditions, and a better ease-of-fibrillatability. The evaluation criteria are as follows (this also applies for fibrillation method B).

+ + : light transmittance of at least 80%

+ : light transmittance of at least 70% but less than 80%

Δ : light transmittance of at least 60% but less than 70%

× : light transmittance of less than 60%

• Fibrillation method B

Each of the oxidized celluloses A to H and P to T obtained as described above was mixed with water to prepare the aqueous oxidized cellulose dispersion having a solids concentration adjusted to 0.1%. This aqueous oxidized cellulose dispersion was introduced into a glass container having a volume of 13.5 mL, and treatment was carried out for 10 minutes using a vortex mixer (VTX-3000L) from LMS to obtain the aqueous CNF dispersion. The solids concentration of each aqueous CNF dispersion was 0.1 mass% in all instances. Each of the aqueous CNF dispersions was introduced into a 10 mm-thick quartz cell, and the light transmittance at a wavelength of 660 nm was measured using a spectrophotometer (JASCO V-550). The measurement values were evaluated using the same four-level scale as for fibrillation method A.

[Slurry viscosity stability]

Water-based slurries (50 g) containing 30 mass% titanium oxide (R-820, Ishihara Sangyo Kaisha, Ltd.) and each particular aqueous CNF dispersion A-H, P-T were prepared, while changing the amount of nanocellulose addition so the initial viscosity, i.e., the viscosity immediately after preparation of the slurry, was the same in each example (300 mPa . s). A "THINKYMIXER ARE-310" mixer from the THINKY Corporation (mix mode, revolution rate = 2,000 rpm, rotation rate = 800 rpm, 20 minutes) was used for the mixing to prepare the water-based slurries.
The viscosity was measured immediately after preparation (initial viscosity) and after standing at quiescence for 1 week, and the percentage change in the viscosity was calculated using the following formula and the viscosity stability of the water-based slurry was evaluated using the evaluation criteria provided below.

$$\text{percentage change in viscosity (\%)} = (N2/N1) \times 100$$

(In the formula, N1 is the initial viscosity of the slurry and N2 is the viscosity of the slurry after standing at quiescence for 1 week after sample preparation.)

+ + : the percentage change in viscosity is less than 105%

+ : the percentage change in viscosity is at least 105% but less than 110%

∆ : the percentage change in viscosity is at least 110% but less than 115%

× : the percentage change in viscosity is at least 115%

Standing at quiescence was carried out in a room (23 ± 2°C).

The initial viscosity of the slurry and its viscosity after standing at quiescence for 1 week were measured using the following conditions: after stirring with a spatula at a speed at which bubbles were not introduced, the measurement was performed using an E-type viscometer (TV-22) from Toki Sangyo Co., Ltd. at 25°C and 100 rpm (shear rate = 200 s$^{-1}$).

[Slurry handling characteristics]

A process liquid was prepared by adding aluminum silicate powder and water to each of the aqueous CNF dispersions A to H and P to T to provide 5 mass% of the aluminum silicate powder and 0.5 mass% nanocellulose, with blending and stirring. This process liquid was lightly stirred with a spatula and then scooped up, and dripping of the liquid when the spatula was tilted was visually observed and the slurry handling characteristics were evaluated using the following criteria.

+ + : Fluid dripping was produced immediately upon tilting.

+ : Fluid dripping was produced from 5 seconds after tilting.

∆ : Fluid dripping was produced from 10 seconds after tilting.

× : Fluid dripping was not produced even after 15 seconds.

[Surface condition after coating with slurry (coating characteristics)]

A process liquid was prepared by adding aluminum silicate powder and water to each of the aqueous CNF dispersions A to H and P to T to provide 5 mass% of the aluminum silicate powder and 0.5 mass% nanocellulose, with blending and stirring. The process liquid was coated on a woven fabric (100% polyester, 100 mm × 100 mm) to provide an aluminum silicate powder application amount of 5 g/m$^2$, and drying was carried out. 10 sheets of the coated woven fabric were visually inspected for nonuniform locations of application (application unevenness), and evaluation was performed using the following criteria.

+ + : Application unevenness was not seen on any of the 10 sheets.

+ : Application unevenness was not seen on 8-9 sheets.

∆ : Application unevenness was not seen on 4 to 7 sheets.

× : Application unevenness was not seen on 1 to 3 sheets, or application unevenness was seen on all 10 sheets.

[Table 1]

| | oxidized cellulose | oxidation method | N-oxyl compound | viscosity-average degree of polymerization | carboxy group content (mmol/g) | average fiber width (nm) | ease-of-fibrillatability | | slurry viscosity stability | slurry handling characteristics | surface condition after coating with slurry |
|---|---|---|---|---|---|---|---|---|---|---|---|
| | | | | | | | fibrillation method A | fibrillation method B | | | |
| Example 1-1 | A | hypochlorite | × | 555 | 0.45 | 4.5 | + | + | + | + | + |
| Example 1-2 | B | | | 455 | 0.61 | 4.2 | + | + | + + | + + | + + |
| Example 1-3 | C | | | 330 | 0.75 | 3.2 | + + | + + | + + | + + | + + |
| Example 1-4 | D | | | 350 | 0.73 | 3.4 | + + | + + | + + | + + | + + |
| Example 1-5 | E | | | 310 | 0.82 | 3.2 | + + | + + | + + | + + | + + |
| Example 1-6 | F | | | 120 | 0.92 | 2.7 | + + | + + | + + | + + | + + |
| Example 1-7 | G | | | 140 | 0.95 | 2.3 | + + | + + | + | + + | + |
| Example 1-8 | H | | | 90 | 0.97 | 2.4 | + + | + + | Δ | + + | Δ |
| Comparative Example 1-1 | P | hypochlorite | × | 730 | 0.42 | 10.3 | × | × | Δ | Δ | Δ |
| Comparative Example 1-2 | Q | | | 650 | 1.12 | 9.7 | × | × | Δ | Δ | Δ |
| Comparative Example 1-3 | R | ozone oxidation | | 610 | 0.43 | 10.9 | × | × | Δ | Δ | Δ |
| Comparative Example 1-4 | S | periodic acid | | 720 | 1.72 | 27.1 | × | × | × | × | × |
| Comparative Example 1-5 | T | TEMPO oxidation | + | 620 | 1.55 | 9.0 | × | × | × | × | × |

In Table 1, an "×" is used to indicate the case in which an N-oxyl compound was not used during the oxidation treatment of the cellulose raw material (i.e., the CNF dispersion substantially did not contain N-oxyl compounds), and a "+" is used to indicate the case in which an N-oxyl compound was used (i.e., an N-oxyl compound was present in the CNF dispersion) (the same applies to Table 2).

For the oxidized celluloses in Examples 1-1 to 1-8, the cellulose microfibrils were readily fibrillated from each other even by mild stirring with a planetary centrifugal mixer or a vortex mixer, and a high light transmittance was exhibited when made into an aqueous CNF dispersion. In addition, a fine cellulose fiber with an average fiber width of not more than 5 nm could be obtained by fibrillation treatment with a planetary centrifugal mixer. Moreover, the viscosity stability, handling characteristics, and coatability were in balance for the slurries of Examples 1-1 to 1-8. In particular, in Examples 1-1 to 1-7, in which the degrees of polymerization were on the order to 3 digit numbers, all of the evaluations of the viscosity stability, handling characteristics, and coatability were "+ +" or "+", and the characteristics of the slurries were thus excellent.

In contrast to this, in Comparative Examples 1-1 and 1-2, in which the degrees of polymerization were 730 and 650, the cellulose microfibrils were resistant to fibrillating therebetween by mild stirring with a planetary centrifugal mixer or vortex mixer and the ease-of-fibrillatability was evaluated as "×". In addition, the slurry characteristics were all evaluated with "Δ" and were worse than in Examples 1-1 to 1-8. The evaluations of the ease-of-fibrillatability and slurry characteristics for Comparative Examples 1-3 to 1-5, in which different oxidation methods were used, were worse than in the examples.

(3) Evaluation 2

[Examples 2-1 to 2-11 and Comparative Examples 2-1 to 2-5]

[Ease-of-fibrillatability]

Using the oxidized celluloses A to H and P to T provided by each of the Production Examples 1 to 8 and Comparative Production Examples 1 to 5, aqueous oxidized cellulose dispersions were prepared having an oxidized cellulose concentration of 0.1% (Examples 2-1, 2-2, 2-4, 2-6, and 2-8 to 2-11, Comparative Examples 2-1 to 2-5) or 0.5% (Examples 2-3, 2-5, and 2-7). The aqueous CNF dispersion obtained by processing the aqueous oxidized cellulose dispersion with a stirrer was introduced into a 10 mm-thick quartz cell, and the light transmittance at a wavelength of 660 nm was measured using a spectrophotometer (JASCO V-550). For the aqueous CNF dispersions obtained by fibrillation of a 0.5% aqueous oxidized cellulose dispersion, the light transmittance was measured after dilution of the aqueous CNF dispersion to 0.1% using pure water. A "THINKYMIXER ARE-310" planetary centrifugal mixer from the THINKY Corporation was used for the stirrer, and a 10-minute treatment was carried out using conditions of a revolution rate of 2,000 rpm and a rotation rate of 800 rpm in mix mode. The evaluation criteria are as follows.

+ + : light transmittance of at least 80%

+ : light transmittance of at least 70% but less than 80%

Δ : light transmittance of at least 60% but less than 70%

× : light transmittance of less than 60%

The average fiber width, slurry viscosity stability, slurry handling characteristics, and surface condition after coating with slurry were measured and evaluated using each of the aqueous CNF dispersions yielded by the fibrillation treatment in the ease-of-fibrillatability evaluation described above. Table 2 gives the results of the evaluations and the carboxy group content of the oxidized celluloses used in the fibrillation treatment. The measurement and evaluation of the average fiber width, slurry viscosity stability, slurry handling characteristics, and surface condition after coating with slurry were carried out as in (2) above.

[Table 2]

| | oxidized cellulose | oxidation method | carboxy group content (mmol/g) | average fiber width (nm) | ease-of-fibrillatability | | | slurry viscosity stability | slurry handling characteristics | surface condition after coating with slurry |
|---|---|---|---|---|---|---|---|---|---|---|
| | | | | | oxidized cellulose concentration during fibrillation (%) | light transmittance (%) | evaluation | | | |
| Example 2-1 | A | hypochlorite | 0.45 | 4.5 | 0.1 | 70.4 | + | + | + | + |
| Example 2-2 | B | | 0.61 | 4.2 | 0.1 | 79.0 | + | + + | + + | + + |
| Example 2-3 | B | | 0.61 | 4.2 | 0.5 | 75.3 | + | + | + | + |
| Example 2-4 | C | | 0.75 | 3.2 | 0.1 | 94.8 | + + | + + | + + | + + |
| Example 2-5 | C | | 0.75 | 3.2 | 0.5 | 90.8 | + + | + + | + + | + + |
| Example 2-6 | D | | 0.73 | 3.4 | 0.1 | 86.7 | + + | + + | + + | + + |
| Example 2-7 | D | | 0.73 | 3.4 | 0.5 | 82.6 | + + | + + | + + | + + |
| Example 2-8 | E | | 0.82 | 3.2 | 0.1 | 83.5 | + + | + + | + + | + + |
| Example 2-9 | F | | 0.92 | 2.7 | 0.1 | 86.7 | + + | + + | + + | + + |
| Example 2-10 | G | | 0.95 | 2.3 | 0.1 | 86.7 | + + | + | + + | + |
| Example 2-11 | H | | 0.97 | 2.4 | 0.1 | 88.2 | + + | Δ | + + | Δ |
| Comparative Example 2-1 | P | hypochlorite | 0.42 | 10.3 | 0.1 | 57.8 | × | Δ | Δ | Δ |
| Comparative Example 2-2 | Q | | 1.12 | 9.7 | 0.1 | 59.6 | × | Δ | Δ | Δ |
| Comparative Example 2-3 | R | ozone oxidation | 0.43 | 10.9 | 0.1 | 55.7 | × | Δ | Δ | Δ |
| Comparative Example 2-4 | S | periodic acid | 1.72 | 27.1 | 0.1 | 51.3 | × | × | × | × |
| Comparative Example 2-5 | T | TEMPO oxidation | 1.55 | 9.0 | 0.1 | 55.0 | × | × | × | × |

For the oxidized celluloses in Examples 2-1 to 2-11, the cellulose microfibrils were readily fibrillated from each other even by mild stirring with a planetary centrifugal mixer, and a high light transmittance was exhibited when made into the aqueous CNF dispersion. In addition, even when the oxidized cellulose concentration during the fibrillation treatment was raised from 0.1% to 0.5%, the light transmittance of the aqueous CNF dispersion was satisfactorily high and the viscosity stability, handling characteristics, and coatability of the water-based slurries were also in balance.

In contrast to this, for all of Comparative Examples 2-1 to 2-5, where fibrillation was carried out by mild stirring with a planetary centrifugal mixer, the light transmittance of the aqueous CNF dispersions were low values, on the level of 50%. In addition, the slurry characteristics in Comparative Examples 2-1 to 2-5 were also inferior to those in Examples 2-1 to 2-11.

**Claims**

1.  An oxidized cellulose that is an oxide of a cellulose raw material by a hypochlorous acid or a salt thereof, wherein the oxidized cellulose

    substantially does not comprise a N-oxyl compound and
    has a degree of polymerization of not more than 600.

2.  The oxidized cellulose according to claim 1, wherein the oxidized cellulose has a carboxy group content of 0.30 to 2.0 mmol/g.

3.  The oxidized cellulose according to claim 1 or 2, wherein an aqueous nanocellulose dispersion obtained by carrying out a fibrillation treatment on an aqueous dispersion of said oxidized cellulose present at a concentration of 0.1 mass% using a planetary centrifugal mixer and conditions of 10 minutes, a revolution rate of 2,000 rpm, and a rotation rate of 800 rpm, has a light transmittance of at least 60%.

4.  An oxidized cellulose that is an oxide of a cellulose raw material, wherein an aqueous nanocellulose dispersion obtained by carrying out a fibrillation treatment on an aqueous dispersion of said oxidized cellulose present at a concentration of 0.1 mass% using a planetary centrifugal mixer and conditions of 10 minutes, a revolution rate of 2,000 rpm, and a rotation rate of 800 rpm, has a light transmittance of at least 60%.

5.  The oxidized cellulose according to claim 4, wherein the oxidized cellulose is an oxide of a cellulose raw material by a hypochlorous acid or a salt thereof.

6.  An oxidized cellulose dispersion in which the oxidized cellulose according to any one of claims 1 to 5 is dispersed in a dispersion medium.

7.  A nanocellulose having an average fiber width of 1 to 200 nm and provided by the fibrillation of the oxidized cellulose according to any one of claims 1 to 6.

8.  A nanocellulose obtained by subjecting the oxidized cellulose according to any one of claims 1 to 6 to a fibrillation treatment for 3 to 15 minutes using a planetary centrifugal mixer at a revolution rate of 1,200 to 2,500 rpm and a rotation rate of 600 to 1,000 rpm.

9.  A nanocellulose dispersion in which the nanocellulose according to claim 7 or 8 is dispersed in a dispersion medium.

## INTERNATIONAL SEARCH REPORT

| International application No. |
| --- |
| **PCT/JP2021/025936** |

### A.    CLASSIFICATION OF SUBJECT MATTER

*C08B 15/04*(2006.01)i; *A61Q 1/00*(2006.01)i; *A61Q 19/00*(2006.01)i; *A61K 9/10*(2006.01)i; *A61K 8/73*(2006.01)i; *A61K 47/38*(2006.01)i
FI:   C08B15/04; A61Q1/00; A61Q19/00; A61K8/73; A61K9/10; A61K47/38

According to International Patent Classification (IPC) or to both national classification and IPC

### B.    FIELDS SEARCHED

Minimum documentation searched (classification system followed by classification symbols)

C08B15/04; A61Q1/00; A61Q19/00; A61K9/10; A61K8/73; A61K47/38

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C.    DOCUMENTS CONSIDERED TO BE RELEVANT

| Category* | Citation of document, with indication, where appropriate, of the relevant passages | Relevant to claim No. |
| --- | --- | --- |
| X | JP 49-034989 A (ASAHI KASEI INDUSTRY CO., LTD.) 30 March 1974 (1974-03-30) examples 1, 2, comparative examples 1, 2 | 1-3, 6-9 |
| Y | | 1-3, 6-9 |
| X | JP 2017-193814 A (KANTO DENKA KOGYO CO., LTD.) 26 October 2017 (2017-10-26) example 1, paragraphs [0032], [0042], claim 1 | 1-3, 6-9 |
| Y | | 1-3, 6-9 |
| X | WO 2020/027307 A1 (TOAGOSEI CO., LTD.) 06 February 2020 (2020-02-06) examples, paragraphs [0036], [0037], claims | 1-3, 6-9 |
| Y | | 1-3, 6-9 |
| X | WO 2018/230354 A1 (TOAGOSEI CO., LTD.) 20 December 2018 (2018-12-20) examples, paragraph [0042] | 1-3, 6-9 |
| Y | | 1-3, 6-9 |
| A | JP 2015-113453 A (THE UNIVERSITY OF TOKYO) 22 June 2015 (2015-06-22) entire text | 1-3, 6-9 |

☐ Further documents are listed in the continuation of Box C.      ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **29 September 2021** | **12 October 2021** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2021/025936**

| Box No. III | Observations where unity of invention is lacking (Continuation of item 3 of first sheet) |
|---|---|

This International Searching Authority found multiple inventions in this international application, as follows:

The claims of this international application are considered to include the following two inventions.

(Invention 1)

Claims 1-3, and parts referring to invention in claims 1-3 in claims 6-9.

Claim 1 is considered to be an invention of a composition of matter pertaining to an oxidized cellulose specified by the matter that the raw material for an oxidizing agent is hypochlorous acid or a salt thereof, and the parameter pertaining to the content and degree of polymerization of an N-oxyl compound.

Meanwhile, document 1 cited in the ISR discloses a method for producing an oxidized cellulose using sodium hypochlorite as an oxidizing agent and not using an N-oxyl compound, indicates that oxidized celluloses each having a degree of polymerization of 85, 83, 180, or 170 were sequentially prepared in examples 1 and 2 and comparative examples 1 and 2, and also indicates that in the treatment process after the preparation of the oxidized cellulose in each example, a dispersion was prepared.

Therefore, claims 1 and 6 lack novelty in light of document 1, and thus the invention in claims 1 and 6 does not have a special technical feature.

Claims 2 and 3, and claims 6-9 referring to any of claims 1-3 are dependent on claim 1 and inventively related to claim 1, and are thus classified as invention 1.

(Invention 2)

Claim 4 cannot be said to share an identical or corresponding special technical feature with claim 1 classified as invention 1.

Claim 4 is considered to be an invention of a composition of matter pertaining to an oxidized cellulose specified by the parameter of the light transmittance of a nanocellulose aqueous dispersion obtained by the predetermined defibration treatment of the present application, and the present invention is not substantially identical or equivalent to claim 1 and any of claims 2 and 3 inventively related to claim 1.

Moreover, the common technical feature shared by the inventions in claim 1 and claim 4 is only an oxidized cellulose, and there are also documents that deprive the invention in claim 1 of novelty, and thus there do not exist special technical features between the inventions in claim 1 and claim 4.

Therefore, claims 4 and 5, and claims 6-9 referring to any of claims 4 and 5 are not related to claim 1, and are thus classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-3, and invention parts referring to claims 1-3 in claims 6-9.**

**Remark on Protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2021/025936**

| Patent document cited in search report | | | Publication date (day/month/year) | Patent family member(s) | | | Publication date (day/month/year) |
|---|---|---|---|---|---|---|---|
| JP | 49-034989 | A | 30 March 1974 | (Family: none) | | | |
| JP | 2017-193814 | A | 26 October 2017 | (Family: none) | | | |
| WO | 2020/027307 | A1 | 06 February 2020 | (Family: none) | | | |
| WO | 2018/230354 | A1 | 20 December 2018 | US 2020/0270369 A1 examples, table 1 EP 3640264 A1 CN 110520448 A | | | |
| JP | 2015-113453 | A | 22 June 2015 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018230354 A

- WO 2020027307 A

**Non-patent literature cited in the description**

- *Sustainable Chem. Eng.,* 2020, vol. 8 (48), 17800-17806